# EUROPEAN PATENT APPLICATION

(11) **EP 2 804 263 A1**
(43) Date of publication of application: **19.11.2014**
(21) Application number: 12865414.2
(22) Date of filing: 16.11.2012
(51) Int. Cl.: H01R 12/71, G01N 33/49, H01R 13/11

(54) **CONNECTOR FOR CONNECTING BIO-SENSOR AND MEASURING INSTRUMENT THEREOF**

(30) Priority: 13.01.2012 KR 20120004331
(71) Applicant: i-Sens, Inc., Nowon-gu Seoul 139-845 (KR)
(72) Inventor: KIM, Moon Hwan, Seoul 130-860 (KR); CHA, Geun Sig, Seoul 120-100 (KR); NAM, Hakhyun, Seoul 142-742 (KR)
(74) Representative: Pearson, Samuel John
(86) International application number: PCT/KR2012/009716
(87) International publication number: WO 2013/105727

(57) **Abstract**

The present invention relates to a connector for connecting a bio-sensor and a measuring instrument thereof, and the connector for connecting a bio-sensor and a measuring instrument thereof according to the present invention comprises an insertion guide which can prevent damage to a connection electrode caused by incorrect insertion of the biosensor into the connector, and the connector further comprises an integrated photo-reflector which emits and detects infrared rays for confirming production lot information of the biosensor, thereby easily confirming production information of the bio-sensor, and thus, the connector for connecting the bio-sensor and the measuring instrument therof is useful.

## Description

### CROSS-REFERENCES TO RELATED APPLICATION

This patent application claims the benefit of priority under 35 U.S.C. § 119 from Korean Patent Application No. 10-2012-0004331 filed 01. 13. 2012, the contents of which are incorporated herein by reference.

### BACKGROUND OF THE INVENTION

### 1. Field of the Invention

The present invention relates to a connector for connecting a biosensor and a measuring device thereof.

### 2. Description of the Related Art

Recently, the need for periodically measuring glucose (blood glucose) in blood has increased in the diagnosis and prophylaxis of diabetes. Nowadays, strip-type biosensors designed for hand-held portable measuring devices allow individuals to readily monitor glucose levels in blood.

Although such electrochemical biosensors are generally conveniently used to monitor and control the amount of blood glucose, accuracy of the biosensors greatly depends on deviations according to each mass-production lot in which the biosensors are produced.

In order to eliminate such a deviation, most commercialized biosensors are designed such that a user directly inputs calibration curve information, which is predetermined at the factory, into a measuring device capable of reading the biosensor. However, such a method is highly inconvenient for the user and causes the user to make input errors, thus leading to inaccurate results.

In order to solve such problems, the inventors of the present application have proposed a biosensor in which production lot information is recorded, and a measuring device capable of automatically identifying production lot information in Korea Patent Application Nos. 2007-0020447 and 2007-0021086, in which production time is short and marking is simple and convenient to maintain mass productivity of the sensor to the maximum by hue marks convenient for printing on a small area of the biosensor and hole marks that may be easily marked at the same time with a last press process of mass production, and production lot information of the sensor may be automatically identified by inputting production lot information to an insulating layer of the biosensor through the marks when a user connects the biosensor to the measuring device so as to measure blood glucose, so that errors that may occur when the user manually inputs information may be prevented and an accurate measurement value may be obtained.

At this time, the marks have been identified by a luminary-detector system reading and calculating the production lot information within the measuring device. However, an operation method of the luminary-detector system requires a process of dispersing and identifying light beams because multiple color light emitting diodes concurrently irradiate light beams onto a production lot information identification unit, and then a detector detects multiple wavelengths of reflected or transmitted light beams, so that a subsequent calculating procedure is complicated, and thus instruments and programs are complicated. Therefore, the related arts have a problem in which costs for configuring the luminary-detector system are increased.

Also, when a biosensor strip is inserted into a connector, there often occurs a case in which a connection electrode pin placed inside the connector is damaged.

In order to solve the above described problems, the inventors have found that the connection electrode pin inside the connector may be prevented from being damaged by providing a guide between an insertion hole of the biosensor to a region where the connection electrode pin starts, in a longitudinal direction from the insertion hole, and also that the production lot information may be simply identified by providing the production lot information identification unit to the biosensor, and by further adding an integrated photo reflector device for emitting and detecting light, thereby completing the present invention.

### SUMMARY OF THE INVENTION

Embodiments of the present invention are directed to provide a connector for connecting a biosensor and a measuring device thereof.

According to an aspect of the present invention, there is provided a connector for electrically connecting a biosensor and a measuring device thereof and including one or more connection electrodes contacting an electrode of the biosensor, the connector including an insertion guide for guiding the biosensor such that the electrode of the biosensor contacts the connection electrode of the connector, wherein the insertion guide is provided to an insertion portion, placed on the same surface as a surface on which the connection electrode is placed, and is shaped with one or more protrusions in which a gap of the insertion portion is narrowed in a direction into which the biosensor is inserted.

### BREIF DESCRIPTION OF THE DRAWINGS

The above and other objects, features and other advantages of the present invention will be more clearly understood from the following detailed description taken in conjunction with the accompanying drawings, in which:
FIG. 1 is an exploded perspective view illustrating a biosensor, in which production lot information indicated by an infrared absorption/reflection mark is recorded on an upper substrate according to an embodiment of the present invention;
FIG. 2 is an exploded perspective view illustrating a biosensor, in which production lot information indicated by an infrared absorption/reflection mark is recorded on a lower substrate according to an embodiment of the present invention;
FIG. 3 is a perspective view illustrating a connector including an insertion guide and a photo reflector device according to an embodiment of the present invention; and
FIG. 4 is a cross-sectional view illustrating a connector including an insertion guide according to an embodiment of the present invention.

### DESCRIPTION OF THE PREFERRED EMBODIMENTS

Features and advantages of the present invention will be more clearly understood by the following detailed description of the present preferred embodiments by reference to the accompanying drawings. It is first noted that terms or words used herein should be construed as meanings or concepts corresponding with the technical sprit of the present invention, based on the principle that the inventor can appropriately define the concepts of the terms to best describe his own invention. Also, it should be understood that detailed descriptions of well-known functions and structures related to the present invention will be omitted so as not to unnecessarily obscure the important point of the present invention.

Hereinafter, specific embodiments of the present invention will be described in detail with reference to the accompanying drawings.

Prior to describing the present invention, the term "biosensor" throughout this specification is used as having the same meaning as the term "biosensor strip".

Hereinafter, the present invention will be described in detail.

The present invention provides a connector for electrically connecting a biosensor and a measuring device thereof and including one or more connection electrodes contacting an electrode of the biosensor, in which the connector includes an insertion guide for guiding the biosensor such that the electrode of the biosensor contacts the connection electrode of the connector, wherein the insertion guide is provided to an insertion portion, placed on the same surface as a surface on which the connection electrode is placed, and is shaped with one or more protrusions in which a gap of the insertion portion is gradually narrowed in a direction into which the biosensor is inserted.

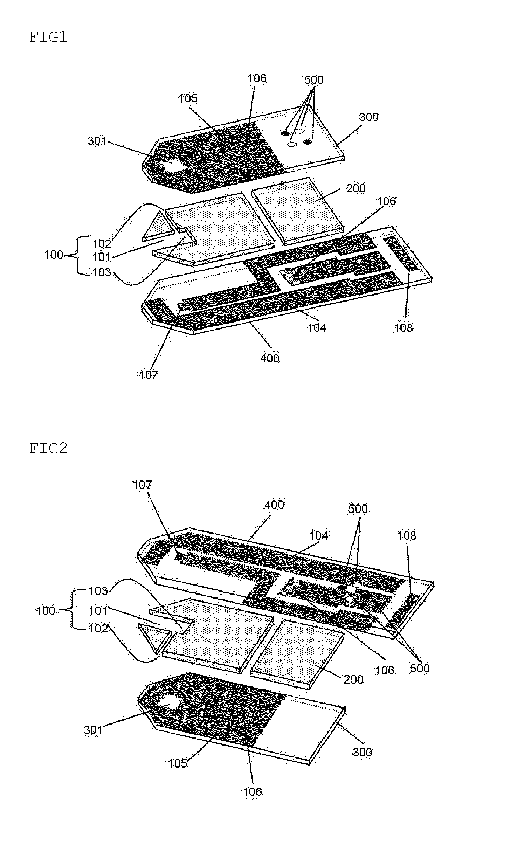

Hereinafter, the connector 700 will be described in detail with reference to FIGS. 1 to 3.

In the connector 700 according to the present invention, the connection electrode 705 plays a role in connecting the biosensor and the measuring device. Specifically, the connection electrode 705 may be placed on an upper surface or a lower surface of the connector 700, and for example, included in the connector 700 in a shape in which a vertex of the connection electrode 705 curved in a V-shape or a U-shape in an inner direction of the connector 700 contacts electrodes of the biosensor.

In an example, the connection electrode 705 may be connected to each of a working electrode 104, an auxiliary electrode 105, a flow sensing electrode 107, and a check electrode 108 of the biosensor.

Here, the flow sensing electrode 107 is an electrode for measuring fluidity of a sample, and the check electrode 108 is an electrode for checking an insertion of the biosensor into the connector 700.

In the connector 700 according to the present invention, the insertion guide 711 guides the biosensor such that the electrodes of the biosensor contact the connection electrode 705 of the connector 700, and prevents the connection electrode 705 from being damaged when the biosensor is forcibly inserted into the connector 700.

Specifically, the insertion guide 711 is provided to a biosensor insertion portion, placed on the same surface as a surface on which the connection electrode 705 is placed, and is shaped with one or more protrusions in which a gap of the insertion portion is narrowed in a direction into which the biosensor is inserted.

At this time, the protrusions may be formed in various shapes, such as a triangle, a circle, an oval, or the like.

Also, the insertion guide 711 is provided at a height lower than that between a surface on which the connection electrode 705 is placed, and a contact point of the connection electrode 705.

When the insertion guide 705 has a height greater than that between the surface and the contact point, there may occur a problem in which the electrodes of the biosensor fail to fully contact the connection electrode 705 of the connector 700.

Furthermore, the insertion guide 711 may be provided so as to correspond to a change of thickness of the biosensor.

For example, the insertion guide 711 may be formed of a material having elasticity, or having a mechanical buffer function.

Meanwhile, the connector 700 according to the present may further include an integrated photo reflector device for emitting and detecting infrared rays on a surface facing a surface on which the connection electrode 705 and the insertion guide 711 are provided, so as to identify a production lot information identification unit 702 of the biosensor.

Hereinafter, a biosensor used in the present invention will be described in detail.

The biosensor used in the present invention includes an electrode unit including a working electrode 104 and an auxiliary electrode 105 formed on any one or both of two planar insulating plates, a microchannel sample introducing unit 100 for introducing a liquid sample into the electrode unit, a reaction reagent layer formed on the working electrode and containing an enzyme and an electron transfer mediator, an electrode connection unit 106 for connecting the working electrode 104 and the auxiliary electrode 105, and a production lot information identification unit 500 in which production lot information is recorded on a portion of at least one of the two planar insulating plates which does not interrupt a connection between the electrodes, wherein the production lot identification unit has several infrared absorption/reflection marks formed by printing a colored or colorless material having a difference in absorbance or reflectance of infrared ray according to a predetermined pattern.

The electrode unit of the biosensor used in the present invention may be formed on any one or both of two planar insulating layers.

That is, (1) one working electrode and one auxiliary electrode may be formed on the same planar insulating plate or (2) the working electrode and the auxiliary electrode may be formed facing each other on the two planar insulating plates [face-to-face electrode; reference: E. K. Bauman et al., Analytical Chemistry, vol 37, p 1378, 1965; K. B. Oldham in "Microelectrodes: Theory and Applications, " Kluwer Academic Publishers, 1991].

Furthermore, the electrode unit of the biosensor used in the present invention may further include a flow sensing electrode 107 which is disposed behind the working electrode 104 and is capable of measuring fluidity of a whole blood sample on the lower planar insulating plate.

Hereinafter, the biosensor will be described in more detail by exemplifying a face-to-face electrode (FIGS. 1 and 2).

In case the biosensor is configured to include a face-to-face electrode, the working electrode 104 and the auxiliary electrode 105 may be formed to have a structure in which the electrodes are isolated by a pressure-adherent isolation plate having a thickness of 50 µm to 250 µm at a location facing each other symmetrically or asymmetrically.

The isolation plate forms a sample cell unit for injecting a bionic sample into a measurement space which is formed by the working electrode 104 and the auxiliary electrode 105, and storing the sample therein, the sample cell unit having a total volume in unit of microliter. The sample cell unit includes a sample introducing portion and a microchannel.

When the electrode is formed, the flow sensing unit in the isolation plate is spaced a predetermined distance apart from the working electrode or the auxiliary electrode. It is preferred that the flow sensing electrode be placed at a distance at which blood that has a globule of 40% and is treated with fluoride can arrive within about 600 ms along the microchannel having a width of 0.5 mm to 2 mm and a height of 50 µm to 250 µm. Preferably, the flow sensing electrode is located at a distance at which a sample that is not treated with fluoride can arrive within 300 ms, more preferably, within 200 ms.

The sample introducing portion enables a blood sample to be introduced into an end of the biosensor. In this case, it is preferred that the sample introducing portion be formed in the shape of 'L', to enable rapid, accurate and convenient introduction of the blood sample.

The sample introducing portion has a structure in which an extra space portion is formed at a location at which a sample introducing path and an air vent intersect each other.

In this specification, the term "intersect each other" means that the sample introducing path and the air vent are not arranged in a straight line but intersect each other at one point.

The extra space portion allows a constant and precise amount of sample to be introduced into the path while the sample is measured, and helps discharging of an excessive amount of sample through the air vent.

Furthermore, the extra space portion may be used as a place where the flow sensing electrode is disposed. When a blood sample is introduced into the sample introducing portion, the blood sample moves to the electrode unit through the microchannel.

In the biosensor used in the present invention, the reaction reagent layer may be formed by merely applying a reagent solution only on the working electrode or on both of the working electrode and the flow sensing electrode. The reaction reagent layer includes an enzyme, such as glucose oxidase, lactate oxidase, or the like, an electron transfer media, a water-soluble polymer, such as cellulose acetate, polyvinyl alcohol, polypyrrol, or the like, a fatty acid having 4 to 20 carbon atoms as an agent for reducing a hematocrit effect, and a lipophilic quaternary ammonium salt.

In the biosensor used in the present invention, an electrode connection unit is designed to be formed on the same plane as the working electrode and the auxiliary electrode connection lines which are formed on the insulating plate. Blood glucose measured by the biosensor of the present invention from results of an electrochemical reaction is provided to the measuring device through the electrode connection unit, and thus may be quantified to a specific blood glucose value.

The biosensor according to the present invention may include a production lot information identification unit 500 for providing calibration curve information on liquid samples having various levels of concentration, the calibration curve information being applied for production lot at the time of producing the biosensor, to a user together with production lot information of the biosensor.

The production lot information identification unit 500 may include several infrared absorption/reflection marks which indicate information on a difference in production lot, and on which a colored or colorless material having a differenc in absorbance or reflectance of infrared ray is printed according a predetermined pattern or a transparent film is attached.

Especially, in the case that the transparent film is used as the infrared absorption/reflection mark, the mark is not conspicuously viewed unlike a hue mark or a bar code that is conspicuously viewed, so that information on production lots of various types of biosensors may be marked without damaging an appearance design of an existing biosensor strip.

At this time, it is preferable that the colored or colorless material or the transparent film absorb an infrared wavelength ranging from 700 nm to 1100 nm.

Also, it is preferable that the number of the infrared ray absorption/reflection marks be adjusted to fall within a range of 1 to 10, and the infrared ray absorption/reflection marks may be placed on any of the upper substrate (FIG. 1) or lower substrate (FIG. 2) as long as the infrared ray absorption/reflection marks do not disturb connections of electrodes 104, 105, 107, and 108 and the electrode connection unit 106 on the biosensor.

An operation mechanism for indentifying the production lot information identification unit 500 using the connector connecting the biosensor and the measuring device thereof will be describes in detail below.

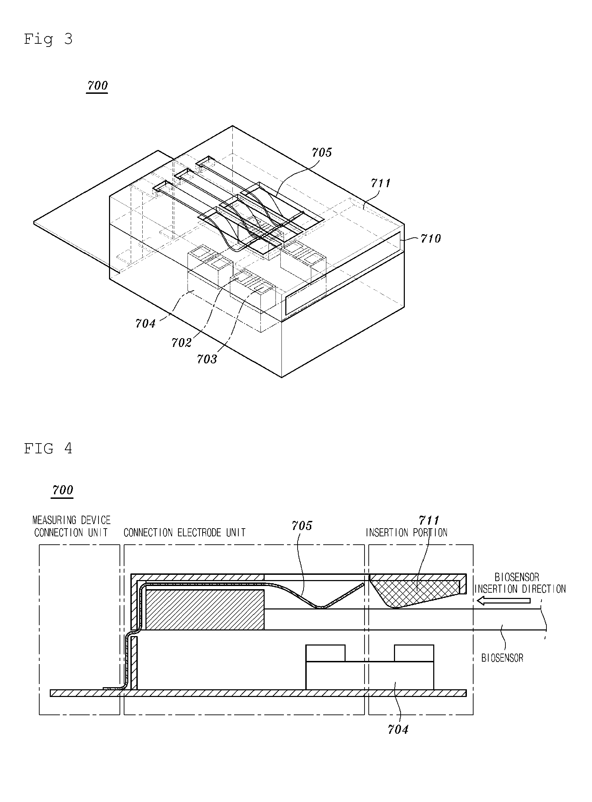

As shown in FIG. 3, a light emitting unit 702 for emitting infrared rays and a light receiving unit 703 are disposed to be oriented in the same direction in the connector, and an integrated photo reflector device is attached to a PCB circuit board 704 having a small space. The production lot information identification unit 500 of the biosensor is detected using the infrared ray emitted or radiated from the photo reflector device. At this time, the light emitting units 702 of the photo reflector device may concurrently or sequentially emit infrared rays.

The infrared rays sensing the production lot information identification unit 500 are absorbed into or reflected from the production lot information identification unit 500, and thus the intensities or wavelengths of the infrared rays vary. The infrared rays reflected as described above are detected by the light receiving unit 703 of the photo reflector unit, for example, a photo diode.

In this case, since the photon reflector device determines whether or not the infrared ray absorption/reflection marks marked on the production lot information identification unit 500 exist, the infrared rays which are emitted from the light emitting unit 702 and have sensed production lot information may be detected by the light receiving unit 703 without a separate filter. Changes in intensity and wavelength of the infrared rays detected by the light receiving unit 703 are identified as digital information of 0 and 1 and transmitted to a measurement operation device, and then the measurement operation device may compares digital information with pre-input information to identify production lot information of the biosensor.

Also, since the photo reflector devices sense the corresponding infrared ray absorption/reflection marks, the number of the photo reflector devices is equal to that of the infrared ray absorption/reflection marks.

Furthermore, the production lot information identification unit 500 marked on the biosensor is not limited to a face-to-face electrochemical biosensor, and may also be applied to a plane type electrochemical biosensor which is implemented such that the working electrode and the auxiliary electrode are formed on the same plate and are thus operated, and a differential type electrochemical biosensor which is implemented such that the face-to-face electrochemical biosensor and the plain type electrochemical biosensor differentially process signals.

The connector according to the present invention may be configured in a structure in which one or more infrared ray absorption or reflection path of light emitting unit-production lot information identification unit-light receiving unit may be acquired, so as to indentify production lot information marked on the biosensor. For example, the connector may be formed of a body having a transparent material, such as transparent acrylic, plastic or the like.

Also, the connector may be provided with a transmission window on any one surface thereof so that infrared rays absorbed or reflected via the light emitting unit-production lot information identification unit-light receiving unit are passed therethrough. Therefore, even when the connector is formed of an opaque material or the body of the connector has a color, the infrared rays irradiated from the light emitting unit may easily reach the production lot information identification unit of the biosensor through the transmission window, and thus production lot information may be identified.

Furthermore, the photo reflector device may be placed inside or outside the connector. In more detail, the photo reflector device may be provided to an upper end portion or a lower end portion of the connector which the biosensor is inserted into and electrically connected to secure the absorption or reflection path of infrared rays, and may be integrally provided inside the connector.

Furthermore, since the photo reflector device may identify 2ⁿ number of infrared ray absorption/reflection marks according to configuration and selection of a device, it may record other variable details including a calibration curve for the production lots, for example, the production time point of the biosensor, whether or not a product of the same manufacturer is used, and whether or not the biosensor is used for a specific model of device. By combining the advantages of such an electrochemical measurement and the advantages of recent small-sized spectroscopic device technologies obtained by the development of technology, a biosensor capable of providing an economical and accurate measurement value may be realized.

A connector for connecting a biosensor and a measuring device thereof according to the present invention may prevent a connection electrode from being damaged due to mis-insertion when the biosensor is inserted into the connector by providing an insertion guide to the connector, and also may further include an integrated photo reflector for emitting and detecting infrared ray in order to identify production lot information of the biosensor, so that production information of the biosensor may be simply opened, and as a result, the connector may be used as a connector for connecting the biosensor and the measuring device thereof.

## Claims

1. A connector for electrically connecting a biosensor and a measuring device thereof and comprising one or more connection electrodes contacting an electrode of the biosensor, the connector comprising an insertion guide for guiding the biosensor such that the electrode of the biosensor contacts the connection electrodes of the connector, wherein the insertion guide is provided to an insertion portion, placed on the same surface as a surface on which the connection electrode is placed, and is shaped with one or more protrusion shapes such that a gap of the insertion portion is narrowed in a direction into which the biosensor is inserted, and provided at a height lower than a height between a surface on which the connection electrode is placed and a contact point of the connection electrode.

2. The connector of claim 1, wherein the insertion guide is provided to correspond to a change in thickness of the biosensor that is being inserted.

3. The connector of claim 1, further comprising an integrated photo reflector device for emitting and detecting an infrared ray to a surface facing a surface on which the connection electrode and the insertion guide are provided, so as to identify a production lot information identification unit of the biosensor.

4. The connector of claim 1, wherein the biosensor comprises,
an electrode unit comprising a working electrode and an auxiliary electrode formed on any one or both of two planar insulating plates;
a microchannel sample introducing unit introducing a liquid sample into the electrode unit;
a reaction reagent layer formed on the working electrode and containing an enzyme and an electron transfer mediator;
an electrode connection unit for connecting the working electrode and the auxiliary electrode; and
a production lot information identification unit in which production lot information is recorded on a portion of at least one of the two planar insulating plates which does not interrupt connections of the electrodes,
wherein the production lot identification unit has several infrared absorption or reflection marks formed by printing a colored or colorless material having a difference in absorbance or reflectance of infrared rays according to a predetermined pattern.

5. The connector of claim 4, wherein the colored or colorless material absorbs a wavelength ranging from 700 nm to 1100 nm.

6. The connector of claim 4, wherein the number of infrared ray absorption or reflection marks is 1 to 10.

7. The connector of claim 3, wherein the biosensor comprises,
an electrode unit comprising a working electrode and an auxiliary electrode formed on any one or both of the two planar insulating plates;
a microchannel introducing unit introducing a liquid sample into the electrode unit;
a reaction reagent layer formed on the working electrode and containing an enzyme and an electron transfer mediator;
an electrode connection unit connecting the working electrode and the auxiliary electrode; and
a production lot information identification unit in which production lot information is recorded on a portion of at least one of the two planar insulating plates which does not interrupt connections of the electrodes,
wherein the production lot identification unit has several infrared absorption or reflection marks formed by printing a colored or colorless material having a difference in absorbance or reflectance of infrared ray according to a predetermined pattern, and
the number of the photo reflector devices is several and is equal to that of the infrared ray absorption/reflection marks printed on the biosensor.

8. The connector of claim 3, wherein the number of the photo reflectors is several, and light emitting units of the several photo reflectors concurrently or sequentially emit light.

9. The connector of claim 3, wherein the photo reflector device has a structure in which one or more infrared ray absorption or reflection paths of light emitting unit-production lot information identification unit-light receiving unit are acquired, so as to indentify production lot information marked on the biosensor.

10. The connector of claim 3, wherein the infrared ray, which is emitted from the light emitting unit to sense the production lot information, is detected by a light receiving unit without a separate filter.

11. The connector of claim 3, wherein the photo reflector device is provided to an upper end portion or a lower end portion of the connector which the biosensor is inserted into and electrically connected to such that the absorption or reflection path of the infrared ray is acquired.
